(19) Europäisches Patentamt
European Patent Office
Office européen des brevets

(11) EP 4 249 039 A1

(12) **EUROPÄISCHE PATENTANMELDUNG**

(43) Veröffentlichungstag:
**27.09.2023 Patentblatt 2023/39**

(21) Anmeldenummer: **22163251.6**

(22) Anmeldetag: **21.03.2022**

(51) Internationale Patentklassifikation (IPC):
***A61M 60/122*** *(2021.01)* ***A61M 60/216*** *(2021.01)*
***A61M 60/422*** *(2021.01)* ***A61M 60/538*** *(2021.01)*
***A61M 60/822*** *(2021.01)*

(52) Gemeinsame Patentklassifikation (CPC):
**A61M 60/178; A61M 60/232; A61M 60/422;
A61M 60/538; A61M 60/822**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder: **Berlin Heart GmbH
12247 Berlin (DE)**

(72) Erfinder:
• **Kiesner, Matthias
15834 Rangsdorf (DE)**

• **Peters, Oliver
14129 Berlin (DE)**
• **Steingräber, Robert
14055 Berlin (DE)**

(74) Vertreter: **Pfenning, Meinig & Partner mbB
Patent- und Rechtsanwälte
Joachimsthaler Straße 10-12
10719 Berlin (DE)**

Bemerkungen:
Der Patentanspruch 16 gilt als fallen gelassen, da die
entsprechende Anspruchsgebühr nicht entrichtet
wurde (R. 45(3) EPÜ).

(54) **STEUEREINHEIT FÜR EINE BLUTPUMPE, PUMPENSYSTEM UND VERFAHREN**

(57) Die Anmeldung betrifft eine Steuereinheit (200) für eine Blutpumpe (300) mit einem magnetisch gelagerten Rotor (320) und einem Stator (340), der eingerichtet ist zum Erzeugen eines Statormagnetfelds zum Ausüben einer Lagerkraft entlang einer Lagerwirkrichtung auf den Rotor (320). Die Blutpumpe (300) ist eingerichtet zum Bereitstellen eines Messsignals, das einen von einer Rotation und/oder Lateralbewegung des Rotors (320) abhängigen Störanteil umfasst. Die Steuereinheit (200) ist dazu eingerichtet, basierend auf dem Messsignal ein von dem Störanteil abhängiges Störsignal zu bestimmen, und basierend auf dem Messsignal und dem bestimmten Störsignal eine Vorgabe für ein Lagersteuersignal derart zu bestimmen, dass der Rotor (320) mittels der Lagerkraft berührungsfrei in dem Gehäuse (301) gelagert wird, wobei eine zum Ausüben der Lagerkraft aufgenommene Leistung gegenüber einer ohne Berücksichtigung des Störsignals bestimmbaren Vorgabe für das Lagersteuersignal reduziert ist,. Die Anmeldung betrifft ferner ein Pumpensystem (100) sowie ein Verfahren zum Ansteuern einer Blutpumpe (200).

Fig. 1

EP 4 249 039 A1

**Beschreibung**

Die Anmeldung betrifft eine Steuereinheit für eine Blutpumpe, insbesondere eine Rotationsfluidpumpe zur Herzunterstützung, ein Pumpensystem mit einer solchen Steuereinheit sowie ein Verfahren zum Ansteuern einer Blutpumpe.

Eine anmeldungsgemäße Blutpumpe umfasst einen in einem Gehäuse magnetisch gelagerten und zum Fördern eines Fluids um eine Rotationsachse rotierbaren Rotor und einen Stator. Der Stator ist eingerichtet zum Erzeugen eines variierbaren Statormagnetfelds zum Ausüben einer gemäß einem Lagersteuersignal variierbaren Lagerkraft entlang einer Lagerwirkrichtung auf den Rotor. Die Blutpumpe ist eingerichtet zum Bereitstellen eines von einer Lagerposition des Rotors entlang der Lagerwirkrichtung abhängigen Messsignals.

Blutpumpen dieser Art sowie entsprechende Steuereinheiten und Ansteuerverfahren sind aus dem Stand der Technik bekannt.

Bei der Ansteuerung einer solchen Blutpumpe kann es vorgesehen sein, basierend auf dem Messsignal eine Vorgabe für das Lagersteuersignal derart zu bestimmen, dass der Rotor mittels der Lagerkraft berührungsfrei entlang der Lagerwirkrichtung in dem Gehäuse gelagert wird, was im Folgenden auch als aktive magnetische Lagerung entlang der Lagerwirkrichtung bezeichnet wird.

Dabei kann das Messsignal neben der Lagerposition auch von weiteren Einflüssen, insbesondere Störungen, abhängen. Diese können beispielsweise von der Erfassung des Messsignals, etwa mittels eines Positionssensors, herrühren. Das auf Grundlage dieses Messsignals bestimmte Lagersteuersignal kann dann einen unerwünschten Anteil umfassen, der für die Lagerung des Rotors nicht erforderlich ist und diese u. U. sogar beeinträchtigen, kann. Durch diesen unerwünschten Anteil können sich die aufgewandte Lagerkraft sowie die damit einhergehende Leistungsaufnahme erhöhen; auch kann das geförderte Blut einer erhöhten Erwärmung ausgesetzt sein.

Vor dem Hintergrund des Stands der Technik liegt der Anmeldung die Aufgabe zugrunde, eine Steuereinheit für eine Blutpumpe, ein Pumpensystem und ein Verfahren zum Ansteuern einer Blutpumpe bereitzustellen, die die genannten Nachteile vermeiden oder verringern.

Zur Lösung der Aufgabe werden eine Steuereinheit nach Anspruch 1, ein Pumpensystem nach Anspruch 12 und ein Verfahren nach Anspruch 16 vorgeschlagen. Vorteilhafte Ausgestaltungen und Weiterbildungen ergeben sich in Verbindung mit den Merkmalen der Unteransprüche.

Die vorgeschlagene Steuereinheit ist eingerichtet zum Ansteuern einer Blutpumpe der oben genannten Art. Die Blutpumpe ist vorzugsweise eingerichtet zum Fördern des Fluids, insbesondere Blut, von einem Einlass zu einem Auslass. Der Einlass und der Auslass können jeweils direkt oder indirekt, insbesondere mittels einer Kanüle, mit einem Herzen und/oder einem Blutgefäß verbindbar sein. Die Blutpumpe kann insbesondere als VAD (ventricular assist device) einsetzbar sein.

Die Blutpumpe ist eingerichtet zum Bereitstellen eines von einer Lagerposition des Rotors entlang der Lagerwirkrichtung abhängigen Messsignals, das einen von einer Rotation des Rotors um die Rotationsache und/oder einer Lateralbewegung des Rotors senkrecht zur Lagerwirkrichtung abhängigen Störanteil umfasst. Das Messsignal kann dabei ein ein- oder mehrdimensionales Messsignal sein, kann also beispielsweise ein einziges Sensorsignal oder mehrere gleichartige oder verschiedene Sensorsignale und/oder davon abgeleitete Signale umfassen. Der Begriff "von einer Lateralbewegung des Rotors senkrecht zur Lagerwirkrichtung abhängig" umfasst dabei auch eine Abhängigkeit von einer Verkippung des Rotors um eine Achse senkrecht zur Rotationsachse X.

Ein Störanteil der genannten Art kann etwa aufgrund von Messfehlern beim Erfassen oder Bereitstellen des Messsignals und/oder aufgrund von Eigenschaften einer zum Erfassen des Messsignals eingerichteten Sensoranordnung auftreten. Insbesondere kann der Störanteil dadurch zustande kommen, dass eine solche Sensoranordnung neben der Lagerposition des Rotors entlang der Lagerwirkrichtung auch die Rotation und/oder Lateralbewegung des Rotors erfasst. Etwa kann die Sensoranordnung ein in Bezug auf die Rotationsachse exzentrisch im oder am Rotor angeordnetes Sensortarget und eine in Bezug auf den Stator fest angeordnete Sensorkomponente umfassen, wobei das Messsignal einem Abstand des Sensortargets von der Sensorkomponente entspricht. Dann kann der Abstand sowohl von der Lagerposition als auch von der Rotation und/oder Lateralbewegung des Rotors abhängen, womit das entsprechende Messsignal einen Störanteil der genannten Art aufweist.

Die Steuereinheit ist dazu eingerichtet, das Messsignal zu erfassen und basierend auf dem in einem Erfassungsintervall erfassten Messsignal ein von dem Störanteil abhängiges Störsignal zu bestimmen und basierend auf dem Messsignal und dem bestimmten Störsignal eine Vorgabe für das Lagersteuersignal derart zu bestimmen, dass der Rotor mittels der Lagerkraft berührungsfrei, insbesondere entlang der Lagerwirkrichtung, in dem Gehäuse gelagert wird, wobei eine zum Ausüben der Lagerkraft aufgenommene Leistung gegenüber einer ohne Berücksichtigung des Störsignals bestimmbaren Vorgabe für das Lagersteuersignal reduziert ist

Das bedeutet insbesondere, dass die zum Ausüben der Lagerkraft aufgenommene Leistung bei einem Ansteuern des Stators gemäß dem Lagersteuersignal entsprechend der unter Berücksichtigung des Störsignals bestimmten Vorgabe geringer ist als eine Leistung, die aufgenommen wird, wenn der Stator gemäß einem Lagersteuersignal angesteuert wird, das einer Vorgabe entspricht, die basierend auf dem Messsignal, aber ohne Bestimmung oder Berücksichtigung des Störsignals bestimmbar ist. Durch das Bestimmen und Berücksichtigen des Störsignals können also die Leistungsaufnahme und damit einhergehende Effekte, etwa eine Erwärmung des geförderten Blutes, verringert werden. Diese Verringerung der Leistungsaufnahme kann insbesondere durch eine Verringerung der aufzubringenden Lagerkraft erreicht werden, die sich dadurch ergibt, dass eine Wirkung des von der Rotation und/oder Lateralbewegung des Rotors abhängigen Störanteils reduziert oder kompensiert wird.

Die Steuereinheit ist ferner dazu eingerichtet, den Stator gemäß dem Lagersteuersignal entsprechend der bestimmten Vorgabe anzusteuern, wobei das Ansteuern zeitlich nach dem Erfassungsintervall erfolgt. Zwischen dem Ende des Erfassungsintervalls und dem Ansteuern des Stators kann ein Verstreichen eines Verzögerungsintervalls vorgesehen sein. Die Steuereinheit ist vorzugsweise dazu eingerichtet, das Erfassen des Messsignals, das Bestimmen des Störsignals, das Bestimmen der Vorgabe für das Lagersteuersignal und das entsprechende Ansteuern des Stators mehrfach wiederholt in einer Abfolge von Ansteuerzyklen durchzuführen. Das Erfassungsintervall und/oder das Verzögerungsintervall eines gegebenen Ansteuerzyklus kann dabei das Erfassungsintervall und/oder das Verzögerungsintervall eines zeitlich darauffolgenden weiteren Ansteuerzyklus wenigstens teilweise überlappen.

Dadurch, dass das Störsignal basierend auf dem im Erfassungsintervall erfassten Messsignal bestimmt und beim Bestimmen der Vorgabe für das Lagersteuersignal berücksichtigt wird, und dadurch, dass das Ansteuern zeitlich nach dem Erfassungsintervall erfolgt, liegt dem Ansteuern ein dem Zeitpunkt des Ansteuerns vorausgegangener Zeitverlauf des Messsignals - und somit des Störanteils - zugrunde. Die Verringerung der Leistungsaufnahme wird also ermöglicht durch ein Berücksichtigen des vorausgegangenen Zeitverlaufs des Messsignals unter der Annahme, dass der Störanteil zum Zeitpunkt des Ansteuerns dem Störanteil zu einem vorangegangenen Zeitpunkt im Wesentlichen entspricht. Unter dieser Annahme kann eine Kompensation für den Störanteil vorgenommen werden, indem das Messsignal und/oder die Vorgabe für das Lagersteuersignal gemäß dem Störsignal korrigiert wird.

Aufgrund dieser Kompensation kann auch ein besonders kompaktes und robustes Design der Blutpumpe selbst möglich sein, da etwa kleinere, kompromissbehaftete Sensoren eingesetzt werden können oder auch eine "sensorlose" Erzeugung des Messsignals, bei der Motorspulenwicklungen des Stators als Sensoren wirken, möglich ist. Auch können Änderungen des Messsignals aufgrund von Beschädigungen, Alterung oder Drift von Komponenten der Blutpumpe ausgeglichen werden, was auch die Lebensdauer und Zuverlässigkeit der Blutpumpe erhöhen kann. Gerade bei der sensorlosen Erzeugung des Messsignals ist ferner die Anzahl von Adern, die in einer Driveline zum Übertragen des Messsignals vorgesehen sein müssen, besonders gering, was ebenfalls ein robustes und kompaktes Design und/oder ein vereinfachtes Vorsehen von Redundanz ermöglichen kann.

Ein Zeitverlauf des Störanteils kann im Wesentlichen periodisch mit einer Periode einer Rotation des Rotors, im Folgenden auch Rotationsperiode, sein. Die Rotationsperiode kann hierbei der mechanischen Rotationsperiode oder, bei Einsatz eines Motors mit mehr als einem magnetischen Polpaar im Rotor zum Antrieb, auch der elektrischen Rotationsperiode des vom Stator erzeugten Magnetfelds entsprechen. Der Zeitverlauf des Störanteils kann einen im Wesentlichen periodischen Anteil und einen gegenüber der Rotationsperiode langsam variierenden Anteil (etwa einen veränderlichen Offset) umfassen. Das Störsignal kann auch von der Rotationsperiode abhängen, also drehzahlabhängig sein.

Im Falle einer Periodizität mit der Rotationsperiode entspricht der Störanteil zu jedem Zeitpunkt des Ansteuerns im Wesentlichen dem Störanteil zu einem vorangegangenen Zeitpunkt, der eine oder mehrere Rotationsperioden zurückliegt.

Dementsprechend kann ein Zeitverlauf des bestimmten Störsignals im Wesentlichen periodisch mit der Rotationsperiode sein. Der Zeitverlauf des Störsignals kann einen im Wesentlichen periodischen Anteil und einen gegenüber der Rotationsperiode langsam variierenden Anteil (etwa einen veränderlichen Offset) umfassen. Das Störsignal kann unter der Randbedingung bestimmt werden, dass es im Wesentlichen periodisch mit der Rotationsperiode ist oder einen im Wesentlichen periodischen Anteil umfasst.

Die Periodizität des Störanteils kann somit ausgenutzt werden, um eine verlässliche Verringerung der Leistungsaufnahme zu erzielen.

Es kann gegeben sein, dass der Störanteil und/oder das Störsignal nicht von der über eine Periode einer Rotation des Rotors gemittelten Lagerposition des Rotors entlang der Lagerwirkrichtung abhängt.

Das Erfassungsintervall kann wenigstens eine, beispielsweise mehrere, Rotationsperioden umfassen, womit eine Bestimmung des Störsignals ermöglichet werden kann.

Die zum Ausüben der Lagerkraft aufgenommene Leistung, die gegenüber einer ohne Berücksichtigung des Störsignals bestimmbaren Vorgabe für das Lagersteuersignal reduziert ist, kann eine zeitlich gemittelte, insbesondere über wenigstens eine Periode der Rotation des Rotors gemittelte, Leistung sein. Eine vorteilhafte Reduktion dieser Leistungsaufnahme kann selbst dann eintreten, wenn die aufzuwendende Lagerkraft nicht zu allen Zeitpunkten verringert (und zu einigen Zeitpunkten u. U. sogar erhöht) wird.

Die Kompensation des Störanteils basierend auf dem bestimmten Störsignal kann an verschiedenen Punkten eingreifen, wie im Folgenden erläutert.

Die Steuereinheit kann einerseits dazu eingerichtet sein, das Lagersteuersignal als korrigiertes Lagersteuersignal zu bestimmen, wobei das bestimmte Störsignal als Kompensationssignal auf ein basierend auf dem Messsignal bestimmtes unkorrigiertes Lagersteuersignal angewandt wird, so dass beim Ansteuern des Stators gemäß dem korrigierten Lagersteuersignal die auf den Rotor ausgeübte Lagerkraft weniger von dem Störanteil des Messsignals beeinflusst wird als bei einem Ansteuern des Stators gemäß dem unkorrigierten Lagersteuersignal.

Andererseits kann - alternativ oder zusätzlich - vorgesehen sein, dass das Messsignal ein unkorrigiertes Messsignal ist und die Steuereinheit dazu eingerichtet ist, beim Bestimmen des Lagersteuersignals ein korrigiertes Messsignal durch Anwenden des bestimmten Störsignal als Kompensationssignal auf das Messsignal zu bestimmen, so dass ein von der Rotation des Rotors um die Rotationsache bzw. der Lateralbewegung des Rotors senkrecht zur Rotationsachse abhängiger Störanteil des korrigierten Messsignals geringer ist als der Störanteil des unkorrigierten Messsignals.

Mittels der gemäß dem Lagersteuersignal variierbaren Lagerkraft ist der Rotor entlang der Lagerwirkrichtung aktiv magnetisch in dem Gehäuse gelagert. Eine Lagerung des Rotors in dem Gehäuse entlang anderer Richtungen kann ebenfalls durch aktive magnetische Lagerung und/oder auf andere Art, etwa durch mechanische, hydrodynamische, hydrostatische und/oder passive magnetische Lagerung erfolgen.

Der Stator und der Rotor können hinsichtlich einer Regelung der Lagerposition des Rotors entlang der Lagerwirkrichtung eine instabile Regelstrecke bilden. Beispielsweise kann die Lage des Rotors entlang der Lagerwirkrichtung destabilisiert werden durch eine passive magnetische Lagerung des Rotors in einer zur Lagerwirkrichtung senkrechten Lateral- oder Radialrichtung. Die Steuereinheit kann zum Stabilisieren der instabilen Regelstrecke mittels eines Lagerreglers eingerichtet sein.

Die Steuereinheit kann dazu eingerichtet sein, das Störsignal mittels einer lernenden Regelung, insbesondere einer iterativ lernenden Regelung und/oder einer repetitiven Regelung und/oder einer Run-to-run-Regelung zu bestimmen. Mittels einer lernenden Regelung kann das Störsignal zuverlässig bestimmt werden, insbesondere wenn der Störanteil periodisch ist bzw. einen periodischen Anteil aufweist; ferner kann auf diese Weise auch eine gegenüber der Rotationsperiode langsame Änderung des Störanteils zuverlässig ausgeglichen werden. Verschiedene Methoden der lernenden Regelung sind beispielsweise beschrieben in: Youqing Wang, Furong Gao, Francis J. Doyle, Survey on iterative learning control, repetitive control, and run-to-run control, Journal of Process Control, Volume 19, Issue 10, 2009, Pages 1589-1600, ISSN 0959-1524, doi:10.1016/j.jprocont.2009.09.006.

Die lernende Regelung kann auf verschiedene Arten, etwa hinsichtlich Stell- und Regelgrößen, implementiert werden. Es kann vorgesehen sein, dass eine Regelgröße der lernenden Regelung eine Ziel-Lagerkraft und eine Stellgröße der lernenden Regelung die Lagerposition des Rotors ist. Es kann vorgesehen sein, dass eine Regelgröße der lernenden Regelung eine Ziel-Lagerkraft und eine Stellgröße der lernenden Regelung die auf den Rotor ausgeübte Lagerkraft ist. Es kann vorgesehen sein, dass eine Regelgröße der lernenden Regelung ein Regelfehler des o. g. Lagerreglers und eine Stellgröße der lernenden Regelung die Lagerposition des Rotors entlang der Lagerwirkrichtung ist.

Die Steuereinheit kann dazu eingerichtet sein, das Lagersteuersignal so zu bestimmen, dass der Rotor in eine Ziel-Lagerposition eingeregelt wird, an der auf den Rotor wirkende äußere Kräfte entlang der Lagerwirkrichtung sich zu einer vorgegebenen Kraft, insbesondere einer Nullkraft, addieren und/oder an der eine zum Erzeugen des variierbaren Statormagnetfelds aufgewandte Leistung minimal ist.

Das vorgeschlagene Pumpensystem umfasst

eine Blutpumpe und eine Steuereinheit der oben vorgeschlagenen Art, wobei die Steuereinheit zum Ansteuern der Blutpumpe eingerichtet ist,
wobei die Blutpumpe einen in einem Gehäuse magnetisch gelagerten und zum Fördern eines Fluids um eine Rotationsachse rotierbaren Rotor und einen Stator umfasst, wobei der Stator eingerichtet ist zum Erzeugen eines variierbaren Statormagnetfelds zum Ausüben einer gemäß einem Lagersteuersignal variierbaren Lagerkraft entlang einer Lagerwirkrichtung auf den Rotor;
wobei die Blutpumpe eingerichtet ist zum Bereitstellen eines von einer Lagerposition des Rotors entlang der Lagerwirkrichtung abhängigen Messsignals, das einen von einer Rotation des Rotors um die Rotationsache und/oder einer Lateralbewegung des Rotors senkrecht zur Lagerwirkrichtung abhängigen Störanteil umfasst.

In dem vorgeschlagenen Pumpensystem entfaltet die vorgeschlagene Steuereinheit ihre oben beschriebenen Vorteile.

Der Rotor kann ein in Bezug auf die Rotationsachse exzentrisch angeordnetes Sensortarget umfassen. Die Blutpumpe kann zum Bereitstellen des Messsignals basierend auf einer Position des Sensortargets eingerichtet sein. Wie weiter oben dargestellt, kann eine Sensoranordnung mit einem exzentrischen Sensortarget einen Störanteil der genannten Art erzeugen. Mittels der vorgeschlagenen Steuereinheit kann diese Störung ausgeglichen und somit etwa eine Designanpassung hin zu einem axial angeordneten bzw. mit der Rotationsachse zusammenfallenden Sensortarget vermieden werden.

Das Messsignal kann einer aufgrund einer Rotation des Rotors induzierten elektromotorischen Gegenkraft

EP 4 249 039 A1

(Back-EMF) entsprechen. Das der Back-EMF entsprechende Messsignal kann ohne zusätzlichen Sensor mittels Motorspulenwicklungen des Stators erfassbar sein, was also eine "sensorlose" Erzeugung des Messsignals ermöglicht.

Die Blutpumpe kann alternativ oder zusätzlich einen Sensor, beispielsweise einen Wirbelstromsensor und/oder einen Magnetfeldsensor, insbesondere einen Hall-Sensor, zum Bereitstellen des Messsignals umfassen.

Die Lagerwirkrichtung kann im Wesentlichen oder annähernd parallel zur Rotationsachse angeordnet sein. Die Lagerwirkrichtung kann in einer anderen Richtung, etwa einer zur Rotationsachse senkrechten Radial- oder Lateralrichtung oder einer zur Rotationsachse in einem anderen Winkel stehenden Richtung, angeordnet sein. Die vorgeschlagene Steuereinheit ist somit mit verschiedenen Pumpen- und Lagertypen verwendbar.

Das vorgeschlagene Verfahren ist vorgesehen zum Ansteuern einer Blutpumpe,

wobei die Blutpumpe einen in einem Gehäuse magnetisch gelagerten und zum Fördern eines Fluids um eine Rotationsachse rotierbaren Rotor und einen Stator umfasst, wobei der Stator eingerichtet ist zum Erzeugen eines variierbaren Statormagnetfelds zum Ausüben einer gemäß einem Lagersteuersignal variierbaren Lagerkraft entlang einer Lagerwirkrichtung auf den Rotor;
wobei die Blutpumpe eingerichtet ist zum Bereitstellen eines von einer Lagerposition des Rotors entlang der Lagerwirkrichtung abhängigen Messsignals, das einen von einer Rotation des Rotors um die Rotationsache und/oder einer Lateralbewegung des Rotors senkrecht zur Lagerwirkrichtung abhängigen Störanteil umfasst.

Das Verfahren umfasst:

Erfassen des Messsignals,
Bestimmen eines von dem Störanteil abhängigen Störsignals basierend auf dem in einem Erfassungsintervall erfassten Messsignal,
Bestimmen, basierend auf dem Messsignal und dem bestimmten Störsignal, einer Vorgabe für das Lagersteuersignal derart, dass der Rotor mittels der Lagerkraft berührungsfrei in dem Gehäuse gelagert wird, wobei eine zum Ausüben der Lagerkraft aufgenommene Leistung gegenüber einer ohne Berücksichtigung des Störsignals bestimmbaren Vorgabe für das Lagersteuersignal reduziert ist, und
zeitlich nach dem Erfassungsintervall erfolgendes Ansteuern des Stators gemäß dem Lagersteuersignal entsprechend der bestimmten Vorgabe.

Das vorgeschlagene Verfahren kann insbesondere unter Verwendung einer Steuereinheit der oben vorgeschlagenen Art und/oder eines Pumpensystems der oben vorgeschlagenen Art ausgeführt und entsprechend den optionalen Merkmalen der Steuereinheit und des Pumpensystems weiter ausgestaltet werden.

Ausführungsbeispiele des Anmeldungsgegenstandes werden nachfolgend anhand von Zeichnungen erläutert. Dabei zeigen:

Fig. 1 eine schematische Darstellung eines Pumpensystems mit einer Blutpumpe und einer Steuereinheit,

Fig. 2 einen Zeitverlauf verschiedener Positionssignale,

Fig. 3 eine schematische Darstellung einer Pumpenregelung,

Fig. 4a bis 4c schematische Darstellungen von Pumpenregelungen gemäß verschiedener Beispiele,

Fig. 5 einen Zeitverlauf einer Zielkraft basierend auf verschiedenen Vorgaben für ein Lagersteuersignal,

Fig. 6a eine Leistungsaufnahme als Funktion einer Rotordrehzahl basierend auf verschiedenen Vorgaben für das Lagersteuersignal,

Fig. 6b eine Regelkraft als Funktion der Rotordrehzahl basierend auf verschiedenen Vorgaben für das Lagersteuersignal.

Wiederkehrende und ähnliche Merkmale verschiedener Ausführungsformen sind in den Abbildungen mit identischen alphanumerischen Bezugszeichen versehen.

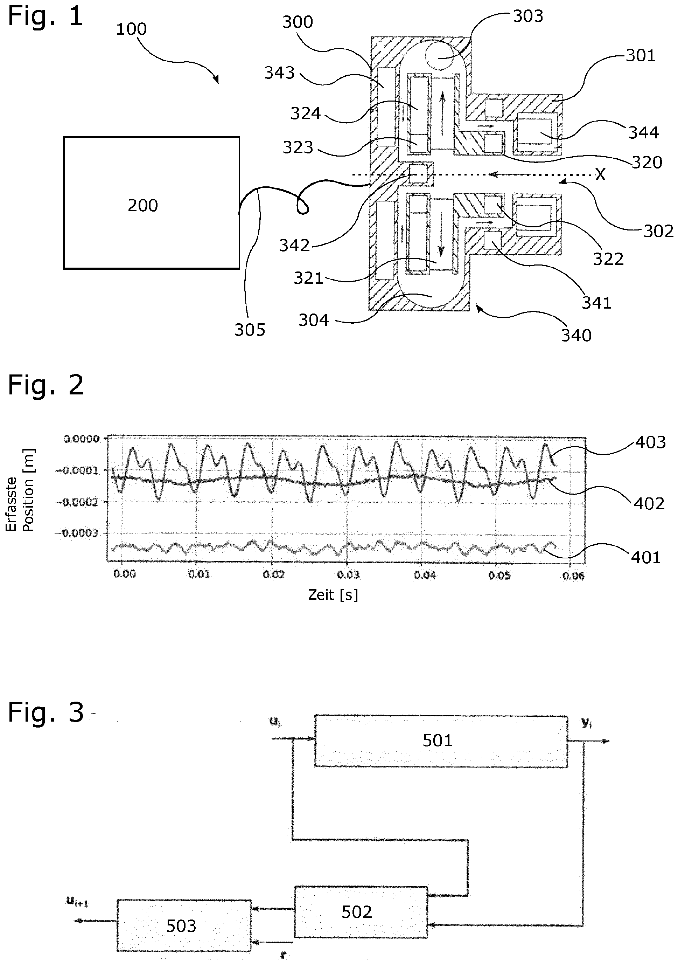

Das in Fig. 1 gezeigte Pumpensystem 100 umfasst eine Blutpumpe 300 und eine Steuereinheit 200, wobei die Steuereinheit 200 zum Ansteuern der Blutpumpe 300 eingerichtet ist.

Die Blutpumpe 300 ist als Rotationsfluidpumpe zur Herzunterstützung ausgebildet und umfasst einen in einem Gehäuse 301 magnetisch gelagerten und zum Fördern eines Fluids, insbesondere Blut, von einem Einlass 302 zu einem Auslass 303 um eine Rotationsachse X rotierbaren Rotor 320 und einen in dem Gehäuse 301 gebildeten Stator 340.

Der Einlass 302 ist im gezeigten Beispiel direkt (durch Implantation in eine Herzwand) mit einem Ventrikel eines Herzens verbindbar, der Auslass 303 ist mittels einer Kanüle, mit einem mit dem Herzen verbundenen Blutgefäß verbindbar. Die Blutpumpe ist somit als VAD einsetzbar.

Der Stator 340 umfasst eine Motorspulenanordnung 343, der Rotor 320 eine Motormagnetanordnung 324, so dass durch Bestromen der Motorspulen ein variierbares Statormagnetfeld erzeugbar ist, mittels dessen der Rotor 320 in Rotation versetzbar ist.

Der Stator 340 ist eingerichtet zum Erzeugen des variierbaren Statormagnetfelds derart, dass eine gemäß einem Lagersteuersignal variierbare Lagerkraft entlang einer Lagerwirkrichtung auf den Rotor 320 wirkt. Entlang der Lagerwirkrichtung, ist der Rotor 320 in dem Gehäuse 301 also mittels der variierbaren Lagerkraft aktiv magnetisch gelagert. Die Motorspulenanordnung 343 und die Motormagnetanordnung 324 wirken hierbei als Lagermagnete. Zusätzlich oder alternativ kann der Stator 340 mindestens eine optionale Steuerspule 344 umfassen, die zum Ausüben der Lagerkraft auf mindestens einen Magneten des Rotors 320, im gezeigten Beispiel auf das einlassseitige Rotormagnetlager 322, wirkt. Das Lagersteuersignal entspricht einer der Motorspulenanordnung 343 und/oder der Steuerspule 344 zu beaufschlagenden Spannung.

Die Lagerwirkrichtung ist im Wesentlichen parallel zur Rotationsachse X angeordnet und wird im Folgenden auch als Axialrichtung bezeichnet. Dazu senkrechte Richtungen werden demensprechend als Radial- bzw. Lateralrichtung bezeichnet. Die Lagerwirkrichtung kann in einer anderen Richtung als der Axialrichtung, etwa in Radial- oder Lateralrichtung oder einer zur Rotationsachse X in einem anderen Winkel stehenden Richtung, angeordnet sein.

Das Fluid tritt im Betrieb im Wesentlichen in Axialrichtung in das Gehäuse 301 und den Rotor 320 ein und wird durch eine Beschaufelung 321 in radialer Richtung in eine Volute 304 des Gehäuses 301 gefördert, von wo aus es den Auslass 303 erreicht. Der Blutfluss ist in Fig. 1 schematisch durch Pfeile verdeutlicht.

Die Blutpumpe 300 des gezeigten Beispiels ist also als Radial- bzw. Zentrifugalpumpe ausgeführt. Der Gegenstand der Anmeldung ist jedoch nicht auf diesen Pumpentyp beschränkt, sondern für verschiedene Arten von Rotationsfluidpumpen anwendbar. Beispielsweise kann die Blutpumpe eine Axialpumpe sein.

Der Rotor 320 ist in dem Gehäuse 301 in allen Raumrichtungen magnetisch gelagert. In radialer Richtung ist der Rotor 320 passiv magnetisch gelagert. Dazu weist der Rotor 320 ein einlassseitiges Rotormagnetlager 322 und ein auslassseitiges Rotormagnetlager 323, der Stator 340 ein einlassseitiges Statormagnetlager 341 und ein auslassseitiges Statormagnetlager 342 auf, wobei das einlassseitige Statormagnetlager 341 auf das einlassseitige Rotormagnetlager 322, das auslassseitige Statormagnetlager 342 auf das auslassseitige Rotormagnetlager 323 wirkt. Wie oben beschrieben, ist der Rotor 321 in axialer Richtung aktiv magnetisch gelagert. Der Gegenstand der Anmeldung ist jedoch nicht auf die beschriebene Lagergeometrie beschränkt, sondern auf verschiedene Pumpen mit aktiver Magnetlagerung entlang wenigstens einer Lagerwirkrichtung anwendbar.

Die Blutpumpe 300 ist eingerichtet ist zum Bereitstellen eines von einer Lagerposition des Rotors 320 entlang der Lagerwirkrichtung abhängigen Messsignals. Die Steuereinheit 200 ist mit der Blutpumpe 300 mittels einer Driveline 305 zum Übertragen des Messsignals und des Lagersteuersignals verbunden.

Das Messsignal entspricht einer aufgrund einer Rotation des Rotors 320 induzierten elektromotorischen Gegenkraft (Back-EMF), die als in der Motorspulenanordnung 343 induzierte Spannung erfassbar ist, was also einer "sensorlose" Erzeugung des Messsignals entspricht. Die Motormagnetanordnung 324 und/oder das einlassseitige Rotormagnetlager 323 wirken hierbei als in Bezug auf die Rotationsachse X exzentrisch angeordnetes Sensortarget. Die Blutpumpe kann alternativ oder zusätzlich einen Sensor, beispielsweise einen Wirbelstromsensor und/oder einen Magnetfeldsensor, insbesondere einen Hall-Sensor, zum Bereitstellen des Messsignals umfassen.

Das Messsignal umfasst einen von einer Rotation des Rotors 320 um die Rotationsache X und einer etwaigen Lateralbewegung des Rotors senkrecht zur Lagerwirkrichtung abhängigen Störanteil, der sich im beschriebenen Fall daraus ergibt, dass die Back-EMF nicht nur von der Lagerposition des Rotors 320 entlang der Lagerwirkrichtung, sondern auch von der Rotation und Lateralbewegung des Rotors 320 abhängt, wobei eine solche Lateralbewegung insbesondere periodisch mit der Rotation einhergehen kann.

Ein Zeitverlauf des Störanteils ist daher im Wesentlichen periodisch mit der Rotationsperiode des Rotors 320, kann dabei allerdings auch einen gegenüber der Rotationsperiode langsam variierenden Anteil umfassen und/oder auch drehzahlabhängig sein. Es können auch periodische Anteile mit Perioden von mehr als einer Rotationsperiode des Rotors 320 auftreten.

Fig. 2 zeigt beispielhaft verschiedene gemessene zeitabhängige Positionssignale 401, 402, 403, die basierend auf verschiedenen, jeweils von der Lagerposition eines Rotors in einer Blutpumpe der beschriebenen Art entlang der Lagerwirkrichtung abhängigen Messsignalen bestimmt wurden. Das Positionssignal 401 basiert auf einem Messsignal, das einer Back-EMF entspricht, wie in der obigen Beschreibung des in Fig. 1 gezeigten Beispiels ausgeführt. Das Positionssignal 402 basiert auf einem Messsignal eines Wirbelstromsensors. Das Positionssignal 403 basiert auf einem Messsignal eines Hall-Sensors. Es ist deutlich erkennbar, dass jedes der Positionssignale 401, 402, 403 einen Anteil mit periodischem Zeitverlauf umfasst. Dieser Anteil rührt von der Rotation des Rotors und ggf. einer mit dieser Rotation

6

assoziierten Lateralbewegung des Rotors her und ist jeweils der zum Ansteuern der aktiven Magnetlagerung relevanten Lagerposition des Rotors überlagert. Der Anteil mit periodischen Zeitverlauf ist daher ein Störanteil im oben genannten Sinne. Dieser hängt nicht oder nur unwesentlich von der über eine Rotationsperiode gemittelten Lagerposition des Rotors entlang der Lagerwirkrichtung ab.

Die in Fig. 1 gezeigte Steuereinheit 200 ist dazu eingerichtet, das Messsignal zu erfassen und basierend auf dem in einem Erfassungsintervall erfassten Messsignal ein von dem Störanteil abhängiges Störsignal zu bestimmen. Das Erfassungsintervall umfasst dabei wenigstens eine, vorzugsweise mehrere, Rotationsperioden. Das Störsignal wird so bestimmt, dass es im Wesentlichen periodisch mit der Rotationsperiode ist.

Ferner ist die Steuereinheit 200 dazu eingerichtet, basierend auf dem Messsignal und dem bestimmten Störsignal eine Vorgabe für das Lagersteuersignal derart zu bestimmen, dass der Rotor 320 mittels der Lagerkraft berührungsfrei in dem Gehäuse 301 gelagert wird, wobei eine zum Ausüben der Lagerkraft aufgenommene Leistung gegenüber einer ohne Berücksichtigung des Störsignals bestimmbaren Vorgabe für das Lagersteuersignal reduziert ist.

Die Steuereinheit 200 ist weiterhin dazu eingerichtet, den Stator 320 gemäß dem Lagersteuersignal entsprechend der bestimmten Vorgabe anzusteuern, wobei das Ansteuern zeitlich nach dem Erfassungsintervall erfolgt. Die Steuereinheit 200 ist dazu eingerichtet, das Erfassen des Messsignals, das Bestimmen des Störsignals, das Bestimmen der Vorgabe für das Lagersteuersignal und das entsprechende Ansteuern des Stators 340 mehrfach wiederholt in einer Abfolge von Ansteuerzyklen durchzuführen.

Die Steuereinheit 200 ist dazu eingerichtet, das Störsignal mittels einer lernenden Regelung zu bestimmen, wie im Folgenden in verschiedenen Beispielen von Pumpenregelungen anhand von Fig. 3 bis Fig. 4c näher erläutert wird.

Für das Bestimmen des Störsignals kommen verschiedene lernende Regelverfahren, insbesondere iterativ lernende Regelung, repetitive Regelung oder Run-to-run-Regelung, in Frage. Da solche Regelverfahren vorteilhaft auf einem stabilen System aufbauen, ist es in dem Beispiel nach Fig. 1 vorgesehen, die aus Stator 340 und Rotor 320 gebildete Regelstrecke mittels eines Lagerreglers, zu stabilisieren. Denn die aus Stator 340 und Rotor 320 gebildete Regelstrecke ist hinsichtlich einer Regelung der Lagerposition des Rotors 320 entlang der Lagerwirkrichtung instabil.

Wie in Fig. 3 schematisch dargestellt ist die Steuereinheit 320 daher zum Stabilisieren dieser instabilen Regelstrecke in einem geschlossenen Regelkreis mittels eines Lagerreglers eingerichtet, wodurch die stabilisierte Regelstrecke 501 gebildet wird. Unabhängig davon, welche Stell- und Regelgrößen für den Lagerregler konkret gewählt werden - hierbei sind, wie weiter unten ausgeführt, verschiedene Optionen möglich -, stellen die Komponenten des Stellgrößenvektors $\mathbf{u}_i$ die Stellgröße für eine Mehrzahl von Zeitschritten eines gegebenen Ansteuerzyklus i, die Kom-ponenten des Stellgrößenvektors $\mathbf{u}_{i+1}$ die Stellgröße für eine Mehrzahl von Zeitschritten des darauf folgenden Ansteuerzyklus i+1, die Komponenten des Regelgrößenvektors $\mathbf{y}_i$ die Regelgröße für die Mehrzahl von Zeitschritten des Ansteuerzyklus i dar. Die stabilisierte Regelstrecke 501 wird mit dem Stellgrößenvektor $\mathbf{u}_i$ beaufschlagt. Zusätzlich werden der Stellgrößenvektor $\mathbf{u}_i$ und der Regelgrößenvektor $\mathbf{y}_i$ in einem Speicher 502 der Steuereinheit 320 gespeichert und einem lernenden Regler 503, im beschriebenen Beispiel einem iterativ lernenden Regler, zugeführt. Der iterativ lernende Regler 503 bestimmt basierend auf dem Stellgrößenvektor $\mathbf{u}_i$ und basierend auf dem Regelgrößenvektor $\mathbf{y}_i$ und basierend auf einem periodischen Referenzvektor $\mathbf{r}$ den Stellgrößenvektor $\mathbf{u}_{i+1}$ für den folgenden Ansteuerzyklus. Dabei kann der periodische Referenzvektor $\mathbf{r}$ beispielsweise auf Null gesetzt werden, wenn ein entsprechendes Ansteuerziel erreicht werden soll. Im folgenden Ansteuerzyklus tritt der Stellgrößenvektor $\mathbf{u}_{i+1}$ an die Stelle des Stellgrößenvektors $\mathbf{u}_i$.

Für eine Auslegung der lernenden Regelung ist ein Modell der Regelstrecke erforderlich (vgl. Gleichungen 1 bis 3), um Vorgaben für eine Korrektur des Regelfehlers bestimmen zu können. Hierbei ist das komplexe dynamische Verhalten der instabilen (stabilisierten) Regelstrecke 501 zu berücksichtigen; dieses wird beispielsweise bei der iterativ lernenden Regelung in Form einer dabei verwendeten quadratischen Lernmatrix berücksichtigt, mit der in jedem Ans-teuerzyklus ein als ($\mathbf{r} - \mathbf{y}_i$) berechneter Regelfehlervektor multipliziert wird. Beispielhafte Verfahren zur Auslegung iterativ lernender Regelungen im Allgemeinen sind etwa in der folgenden Druckschrift angegeben und werden daher hier nicht im Einzelnen wiederholt: Douglas A. Bristow, Marina Tharayil, Andrew G. Alleyne, Survey Of Iterative Learning Control : A Learning-Based Method for High-Performance Tracking Control, IEEE Control Systems, Volume 26, Issue 3, Pages 96-114, 2006, ISSN 1066-033X, doi: 10.1109/MCS.2006.1636313.

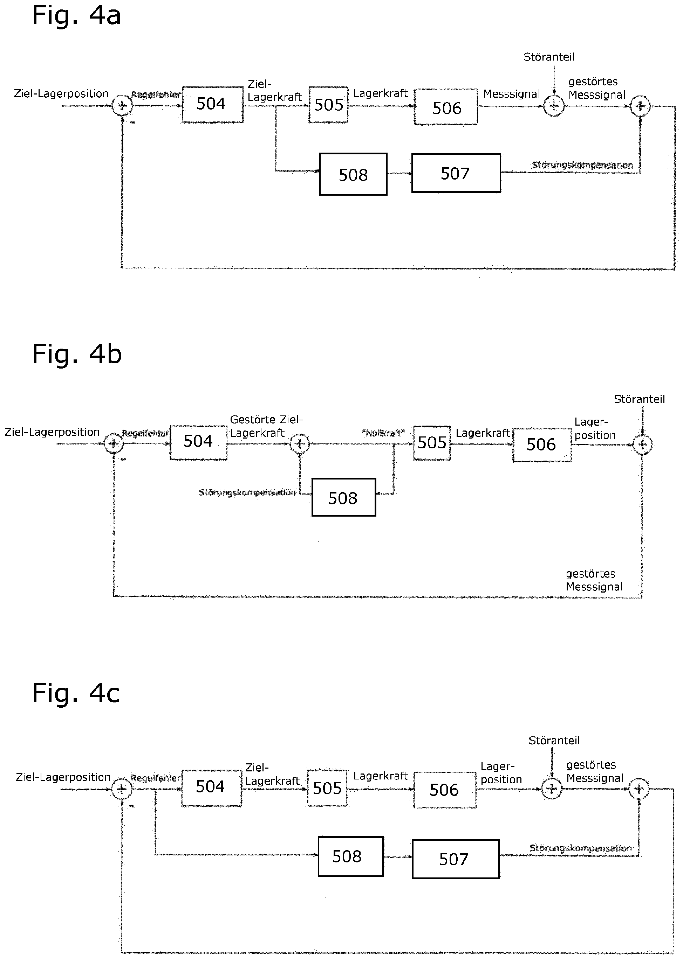

Die Steuereinheit 200 kann ferner dazu eingerichtet sein, ein Starten der Blutpumpe, also ein Hochfahren der Rotation des Rotors 320 vom Stillstand zu einer Ziel-Rotordrehzahl, unter Verwendung der lernenden Regelung durchzuführen, indem zunächst der Rotor mit einer Start-Rotordrehzahl, die geringer ist als die Ziel-Rotordrehzahl, betrieben wird und sodann die folgenden Schritte bis zum Erreichen der Ziel-Rotordrehzahl wiederholt werden: Bestimmen des Störsignals mittels der lernenden Regelung und Störungskompensation wie weiter unten (im Zusammenhang mit Fig. 4a bis 4c) beschrieben, Erhöhen der Rotordrehzahl um ein Drehzahlinkrement.

Unter der Annahme einer linearen Übertragungsfunktion des Lagerreglers von der Messgröße (insbesondere der Lagerposition) zur Stellgröße (insbesondere der Lagerkraft) kann die lernende Regelung im Spektralbereich durchgeführt werden, d. h. die Steuereinheit 200 kann dazu eingerichtet sein, das Störsignal in eine Mehrzahl von Frequenzkomponenten zu zerlegen und die Störungskompensation für jede der Frequenzkomponenten unabhängig durchzuführen. Die Steuereinheit 200 kann ferner dazu eingerichtet sein, eine Kompensationsgröße für mindestens eine - insbesondere die größte -, mehrere oder alle Frequenzkomponenten des Störsignals mittels eines Optimierungsverfahrens, beispielsweise des Newtonschen Verfahrens oder des Gradient-Descent-Verfahrens, zu optimieren und/oder unter Verwendung einer Übertragungsfunktion des Lagerreglers im Spektralbereich einzuregeln.

Für die lernende Regelung können verschiedene Kombinationen von Stell- und Regelgrößen vorgesehen sein. Beispiele für entsprechende Pumpenregelungen sind in Fig. 4a bis Fig. 4c gezeigt. In diesen Beispielen kann der lernende Regler 508 ein iterativ lernender Regler oder ein lernender Regler anderer Art, insbesondere ein repetitiver Regler oder ein Run-to-run-Regler sein.

Bei der in Fig. 4a gezeigten Pumpenregelung ist vorgesehen, dass eine Regelgröße der lernenden Regelung eine Ziel-Lagerkraft und eine Stellgröße der lernenden Regelung die Lagerposition des Rotors 320 ist. Die Regelstrecke für den lernenden Regler 508 ist die Übertragungsfunktion $G_{lc}$ (s) des geschlossenen Regelkreises, umfassend den Lagerregler 504 und das System aus Stator 340 und Rotor 320, welche sich darstellen lässt als

$$G_{lc}(s) = -C(s) / (1 + G(s) C(s)) \qquad \text{(Gleichung 1)},$$

wobei C (s) eine Übertragungsfunktion des Lagerreglers 504 und G (s) eine Übertragungsfunktion der Regelstrecke bestehend aus Stator 340 und Rotor 320 ist. Die Regelstrecke aus Stator 340 und Rotor 320 ist in Fig. 4a bis 4c zerlegt in die Regelungskomponenten Aktor 505 und Pumpenprozessglied 506 dargestellt.

Im Beispiel gemäß Fig. 4a bestimmt der lernende Regler 508 eine geschätzte Sensorstörung 507 als Störsignal. Das vom Pumpenprozessglied 506 ausgegebene Messsignal ist hierbei ein unkorrigiertes Messsignal und die Steuereinheit 200 dazu eingerichtet, beim Bestimmen des Lagersteuersignals ein korrigiertes Messsignal durch Anwenden des bestimmten Störsignals als Kompensationssignal auf das Messsignal zu bestimmen (in der Zeichnung als Störungskompensation bezeichnet), etwa durch Addition des inversen Störsignals. Somit ist ein von der Rotation des Rotors 320 um die Rotationsache X bzw. der Lateralbewegung des Rotors 320 senkrecht zur Rotationsachse X abhängiger Störanteil des korrigierten Messsignals geringer als der Störanteil des unkorrigierten Messsignals.

Bei der in Fig. 4b gezeigten Pumpenregelung ist vorgesehen, dass eine Regelgröße der lernenden Regelung eine Ziel-Lagerkraft und eine Stellgröße der lernenden Regelung die auf den Rotor320 ausgeübte Lagerkraft ist. In diesem Fall ist eine Darstellung der Übertragungsfunktion $G_{lc}$ (s) des geschlossenen Regelkreises gegeben durch

$$G_{lc}(s) = 1 / (1 + G(s) C(s)) \qquad \text{(Gleichung 2)}.$$

Die Steuereinheit 200 ist in diesem Beispiel dazu eingerichtet, das Lagersteuersignal so zu bestimmen, dass der Rotor 320 in eine Ziel-Lagerposition eingeregelt wird, an der auf den Rotor 320 wirkende äußere Kräfte entlang der Lagerwirkrichtung sich zu einer vorgegebenen Kraft, insbesondere einer Nullkraft, addieren.

Die Steuereinheit 200 ist dazu eingerichtet, das Lagersteuersignal als korrigiertes Lagersteuersignal zu bestimmen, wobei in diesem Fall bei der Störungskompensation das durch den lernenden Regler 508 bestimmte Störsignal als Kompensationssignal auf ein basierend auf dem Messsignal bestimmtes unkorrigiertes Lagersteuersignal (gestörte Ziel-Lagerkraft, die von dem Lagerregler 504 ausgegeben wird) angewandt wird. Beim Ansteuern des Stators 340 gemäß dem korrigierten Lagersteuersignal wird somit die auf den Rotor 320 ausgeübte Lagerkraft weniger von dem Störanteil des Messsignals beeinflusst als bei einem Ansteuern des Stators 340 gemäß dem unkorrigierten Lagersteuersignal.

Bei der in Fig. 4c gezeigten Pumpenregelung ist vorgesehen, dass eine Regelgröße der lernenden Regelung ein Regelfehler des Lagerreglers und eine Stellgröße der lernenden Regelung die Lagerposition des Rotors 320 entlang der Lagerwirkrichtung ist. In diesem Fall ist eine Darstellung der Übertragungsfunktion $G_{lc}$ (s) des geschlossenen Regelkreises gegeben durch

$$G_{lc}(s) = -1 / (1 + G(s) C(s)) \qquad \text{(Gleichung 3)}.$$

Wie im Beispiel gemäß Fig. 4a bestimmt auch im Beispiel gemäß Fig. 4b der lernende Regler eine geschätzte Sensorstörung 507 als Störsignal. Das vom Pumpenprozessglied 506 ausgegebene Messsignal ist hierbei wiederum ein unkorrigiertes Messsignal und die Steuereinheit 200 dazu eingerichtet, beim Bestimmen des Lagersteuersignals ein korrigiertes Messsignal durch Anwenden des bestimmten Störsignals als Kompensationssignal auf das Messsignal zu bestimmen (in der Zeichnung als Störungskompensation bezeichnet). Somit ist ein von der Rotation des Rotors 320 um die Rotationsache X bzw. der Lateralbewegung des Rotors 320 senkrecht zur Rotationsachse X abhängiger Störanteil des korrigierten Messsignals geringer als der Störanteil des unkorrigierten Messsignals.

Wie den vorangehenden Ausführungen zu entnehmen ist, eignet sich die Steuereinheit 200 gemäß der vorstehend beschriebenen Beispiele insbesondere zum Ausführen eines Verfahrens zum Ansteuern der Blutpumpe 300, wobei das Verfahren umfasst:

Erfassen des Messsignals,

Bestimmen des von dem Störanteil abhängigen Störsignals basierend auf dem im Erfassungsintervall erfassten Messsignal,

Bestimmen, basierend auf dem Messsignal und dem bestimmten Störsignal, der Vorgabe für das Lagersteuersignal derart, dass der Rotor 320 mittels der Lagerkraft berührungsfrei in dem Gehäuse 301 gelagert wird, wobei eine zum Ausüben der Lagerkraft aufgenommene Leistung gegenüber einer ohne Berücksichtigung des Störsignals bestimmbaren Vorgabe für das Lagersteuersignal reduziert ist, und

zeitlich nach dem Erfassungsintervall erfolgendes Ansteuern des Stators 340 gemäß dem Lagersteuersignal entsprechend der bestimmten Vorgabe.

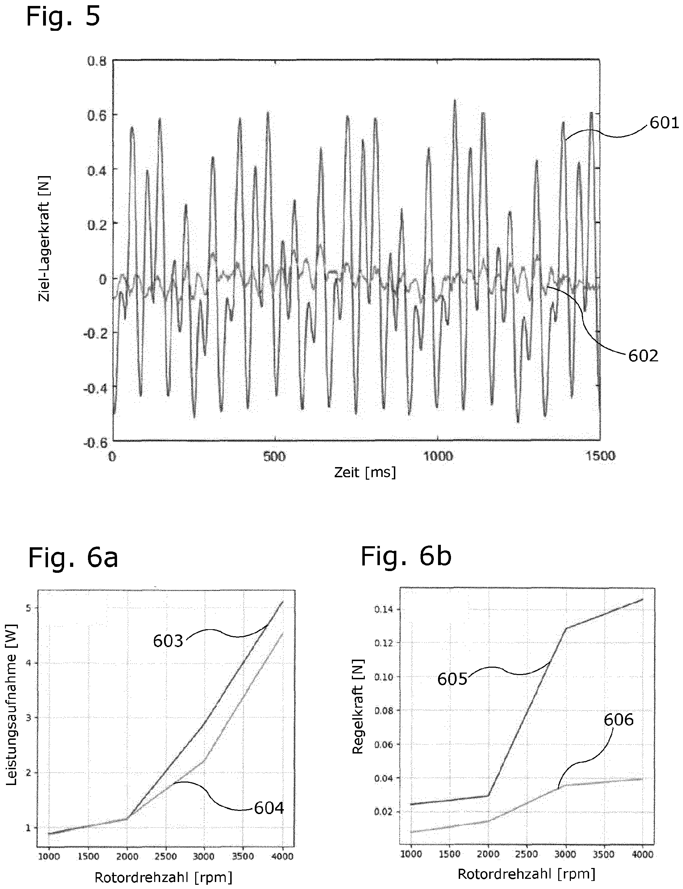

Bestimmte Aspekte einer Verwendung der Steuereinheit 200 und/oder des Pumpensystems 100 und/oder des beschriebenen Verfahrens werden im Folgenden anhand von Fig. 5 bis Fig. 6b am Beispiel der iterativ lernenden Regelung exemplarisch hervorgehoben.

Fig. 5 zeigt gemessene zeitabhängige Ziel-Lagerkraftsignale 601, 602, wobei die Ziel-Lagerkraft eine Stellgröße der Lagerregelung ist. Das zeitabhängige Ziel-Lagerkraftsignal 601 entspricht einer basierend auf dem Messsignal ohne Berücksichtigung des Störsignals bestimmten Vorgabe für das Lagersteuersignal, das zeitabhängige Ziel-Lagerkraftsignal 602 entspricht einer basierend auf dem Messsignal und dem Störsignal bestimmten Vorgabe für das Lagerkraftsignal, wobei letztere unter Verwendung einer iterativ lernen Regelung wie oben beschrieben bestimmt wurde.

In Fig. 5 ist zu erkennen, dass die Ziel-Lagerkraft entsprechend dem Ziel-Lagerkraftsignal 602 Schwankungen von geringerer Amplitude umfasst als die Ziel-Lagerkraft entsprechend dem Ziel-Lagerkraft-Signal 601. In beiden Fällen wurde der Rotor 320 über den Zeitverlauf der Messung mittels der Lagerregelung berührungsfrei entlang der Lagerwirkrichtung in dem Gehäuse 301 gelagert. Demnach ist eine zum Ausüben der Lagerkraft aufgenommene Leistung, insbesondere eine über mehrere Rotationsperioden zeitlich gemittelte Leistung, bei einem Ansteuern des Stators 340 gemäß dem Lagersteuersignal entsprechend der unter Berücksichtigung des Störsignals bestimmten Vorgabe geringer als eine Leistung, die aufgenommen wird, wenn der Stator 340 gemäß einem Lagersteuersignal angesteuert wird, das einer Vorgabe entspricht, die basierend auf dem Messsignal, aber ohne Bestimmung oder Berücksichtigung des Störsignals bestimmbar ist.

Weitere Wirkungen der lernenden Regelung sind in Fig. 6a und Fig. 6b erkennbar. Fig. 6a zeigt Leistungsaufnahmekurven 603, 604, die die aufgenommene Leistung der Blutpumpe 300 als Funktion einer Rotordrehzahl des Rotors 320 darstellen. Analog der im Zusammenhang mit Fig. 5 beschriebenen Situation zeigt die Leistungsaufnahmekurve 603 die aufgenommene Leistung bei Bestimmen der Vorgabe für das Lagersteuersignal ohne Berücksichtigung des Störsignals, die Leistungsaufnahmekurve 604 die aufgenommene Leistung bei Bestimmen der Vorgabe für das Lagersteuersignal mit Berücksichtigung des Störsignals unter Verwendung einer iterativ lernenden Regelung.

Fig. 6b zeigt Regelkraftkurven 605, 606, die ein quadratisches Mittel der für die aktive magnetische Lagerung des Rotors 320 erforderlichen Lagerkraft als Funktion der Rotordrehzahl des Rotors 320 darstellen. Analog der im Zusammenhang mit Fig. 5 beschriebenen Situation zeigt die Regelkraftkurve 605 die erforderliche Lagerkraft bei Bestimmen der Vorgabe für das Lagersteuersignal ohne Berücksichtigung des Störsignals, die Regelkraftkurve 606 die erforderliche Lagerkraft bei Bestimmen der Vorgabe für das Lagersteuersignal mit Berücksichtigung des Störsignals unter Verwendung einer iterativ lernenden Regelung.

Liste der Bezugzeichen:

| | |
|---|---|
| 100 | Pumpensystem, |
| 200 | Steuereinheit, |
| 300 | Blutpumpe, |
| 301 | Gehäuse, |
| 302 | Einlass, |
| 303 | Auslass, |
| 304 | Volute, |
| 305 | Driveline, |
| 320 | Rotor, |
| 321 | Beschaufelung, |
| 322 | einlassseitiges Rotormagnetlager, |
| 323 | auslassseitiges Rotormagnetlager, |

| | |
|---|---|
| 324 | Motormagnetanordnung, |
| 340 | Stator, |
| 341 | einlassseitiges Statormagnetlager, |
| 342 | auslassseitiges Statormagnetlager, |
| 343 | Motorspulenanordnung, |
| 344 | Steuerspule, |
| 401, 402, 403 | zeitabhängiges Positionssignal, |
| 501 | stabilisierte Regelstrecke, |
| 502 | Speicher, |
| 503 | iterativ lernender Regler, |
| 504 | Lagerregler, |
| 505 | Aktor, |
| 506 | Pumpenprozessglied, |
| 507 | geschätzte Sensorstörung, |
| 508 | lernender Regler, |
| 601, 602 | zeitabhängiges Ziel-Lagerkraftsignal, |
| 603,604 | Leistungsaufnahmekurve, |
| 605,606 | Regelkraftkurve, |
| X | Rotationsachse. |

**Patentansprüche**

1. Steuereinheit (200) für eine Blutpumpe (300),

   wobei die Blutpumpe (300) einen in einem Gehäuse (301) magnetisch gelagerten und zum Fördern eines Fluids um eine Rotationsachse (X) rotierbaren Rotor (320) und einen Stator (340) umfasst, wobei der Stator (340) eingerichtet ist zum Erzeugen eines variierbaren Statormagnetfelds zum Ausüben einer gemäß einem Lagersteuersignal variierbaren Lagerkraft entlang einer Lagerwirkrichtung auf den Rotor (320);
   wobei die Blutpumpe (300) eingerichtet ist zum Bereitstellen eines von einer Lagerposition des Rotors (320) entlang der Lagerwirkrichtung abhängigen Messsignals, das einen von einer Rotation des Rotors (320) um die Rotationsache (X) und/oder einer Lateralbewegung des Rotors (320) senkrecht zur Lagerwirkrichtung abhängigen Störanteil umfasst;
   wobei die Steuereinheit (200) dazu eingerichtet ist,

   das Messsignal zu erfassen und basierend auf dem in einem Erfassungsintervall erfassten Messsignal ein von dem Störanteil abhängiges Störsignal zu bestimmen,
   basierend auf dem Messsignal und dem bestimmten Störsignal eine Vorgabe für das Lagersteuersignal derart zu bestimmen, dass der Rotor (320) mittels der Lagerkraft berührungsfrei in dem Gehäuse (301) gelagert wird, wobei eine zum Ausüben der Lagerkraft aufgenommene Leistung gegenüber einer ohne Berücksichtigung des Störsignals bestimmbaren Vorgabe für das Lagersteuersignal reduziert ist, und
   den Stator (340) gemäß dem Lagersteuersignal entsprechend der bestimmten Vorgabe anzusteuern, wobei das Ansteuern zeitlich nach dem Erfassungsintervall erfolgt.

2. Steuereinheit (200) nach Anspruch 1, wobei ein Zeitverlauf des Störanteils und/oder des Störsignals im Wesentlichen periodisch mit einer Periode einer Rotation des Rotors (320) ist.

3. Steuereinheit (200) nach einem der vorhergehenden Ansprüche, wobei der Störanteil und/oder das Störsignal nicht von der über eine Periode einer Rotation des Rotors (320) gemittelten Lagerposition des Rotors (320) entlang der Lagerwirkrichtung abhängt.

4. Steuereinheit (200) nach einem der vorhergehenden Ansprüche, wobei das Erfassungsintervall wenigstens eine Periode der Rotation des Rotors (320) umfasst und/oder wobei die zum Ausüben der Lagerkraft aufgenommene Leistung, die gegenüber einer ohne Berücksichtigung des Störsignals bestimmbaren Vorgabe für das Lagersteuersignal reduziert ist, eine zeitlich gemittelte, insbesondere über wenigstens eine Periode der Rotation des Rotors (320) gemittelte, Leistung ist.

5. Steuereinheit (200) nach einem der vorhergehenden Ansprüche, wobei der Stator (340) und der Rotor (320) hinsichtlich einer Regelung der Lagerposition des Rotors (320) entlang der Lagerwirkrichtung eine instabile Regelstrecke bilden.

6. Steuereinheit (200) nach Anspruch 5, wobei die Steuereinheit (200) zum Stabilisieren der instabilen Regelstrecke mittels eines Lagerreglers eingerichtet ist.

7. Steuereinheit (200) nach einem der vorhergehenden Ansprüche, dazu eingerichtet, das Störsignal mittels einer lernenden Regelung (503, 508), insbesondere einer iterativ lernenden Regelung (503, 508) und/oder einer repetitiven Regelung und/oder einer Run-to-run-Regelung zu bestimmen.

8. Steuereinheit (200) nach Anspruch 7,

   wobei eine Regelgröße der lernenden Regelung (503, 508) eine Ziel-Lagerkraft und eine Stellgröße der lernenden Regelung (503, 508) die Lagerposition des Rotors (320) ist,
   und/oder wobei eine Regelgröße der lernenden Regelung (503, 508) eine Ziel-Lagerkraft und eine Stellgröße der lernenden Regelung (503, 508) die auf den Rotor (320) ausgeübte Lagerkraft ist,
   und/oder wobei eine Regelgröße der lernenden Regelung (503, 508) ein Regelfehler des Lagerreglers und eine Stellgröße der lernenden Regelung (503, 508) die Lagerposition des Rotors (320) entlang der Lagerwirkrichtung ist.

9. Steuereinheit (200) nach einem der vorhergehenden Ansprüche, dazu eingerichtet, das Lagersteuersignal als korrigiertes Lagersteuersignal zu bestimmen, wobei das bestimmte Störsignal als Kompensationssignal auf ein basierend auf dem Messsignal bestimmtes unkorrigiertes Lagersteuersignal angewandt wird, so dass beim Ansteuern des Stators (340) gemäß dem korrigierten Lagersteuersignal die auf den Rotor (320) ausgeübte Lagerkraft weniger von dem Störanteil des Messsignals beeinflusst wird als bei einem Ansteuern des Stators (340) gemäß dem unkorrigierten Lagersteuersignal.

10. Steuereinheit (200) nach einem der vorhergehenden Ansprüche, wobei das Messsignal ein unkorrigiertes Messsignal ist und die Steuereinheit (200) dazu eingerichtet ist, beim Bestimmen des Lagersteuersignals ein korrigiertes Messsignal durch Anwenden des bestimmten Störsignal als Kompensationssignal auf das Messsignal zu bestimmen, so dass ein von der Rotation des Rotors (320) um die Rotationsache (X) bzw. der Lateralbewegung des Rotors (320) senkrecht zur Rotationsachse (X) abhängiger Störanteil des korrigierten Messsignals geringer ist als der Störanteil des unkorrigierten Messsignals.

11. Steuereinheit (200) nach einem der vorhergehenden Ansprüche, wobei die Steuereinheit (200) dazu eingerichtet ist, das Lagersteuersignal so zu bestimmen, dass der Rotor (320) in eine Ziel-Lagerposition eingeregelt wird, an der auf den Rotor (320) wirkende äußere Kräfte entlang der Lagerwirkrichtung sich zu einer vorgegebenen Kraft, insbesondere einer Nullkraft, addieren und/oder an der eine zum Erzeugen des variierbaren Statormagnetfelds aufgewandte Leistung minimal ist.

12. Pumpensystem (100), umfassend
    eine Blutpumpe (300),

    wobei die Blutpumpe (300) einen in einem Gehäuse (301) magnetisch gelagerten und zum Fördern eines Fluids um eine Rotationsachse (X) rotierbaren Rotor (320) und einen Stator (340) umfasst, wobei der Stator (340) eingerichtet ist zum Erzeugen eines variierbaren Statormagnetfelds zum Ausüben einer gemäß einem Lagersteuersignal variierbaren Lagerkraft entlang einer Lagerwirkrichtung auf den Rotor (320);
    wobei die Blutpumpe (300) eingerichtet ist zum Bereitstellen eines von einer Lagerposition des Rotors (320) entlang der Lagerwirkrichtung abhängigen Messsignals, das einen von einer Rotation des Rotors (320) um die Rotationsache (X) und/oder einer Lateralbewegung des Rotors (320) senkrecht zur Lagerwirkrichtung abhängigen Störanteil umfasst;

    eine Steuereinheit (200) gemäß einem der vorhergehenden Ansprüche, eingerichtet zum Ansteuern der Blutpumpe (300).

13. Pumpensystem (100) nach Anspruch 12, wobei der Rotor (320) ein in Bezug auf die Rotationsachse (X) exzentrisch angeordnetes und/oder nicht rotationssymmetrisches Sensortarget umfasst und die Blutpumpe (300) zum Bereitstellen des Messsignals basierend auf einer Position des Sensortargets eingerichtet ist.

**14.** Pumpensystem (100) nach Anspruch 12 oder 13, wobei das Messsignal einer aufgrund einer Rotation des Rotors (320) induzierten elektromotorischen Gegenkraft (Back-EMF) entspricht und/oder wobei die Blutpumpe (300) einen Sensor, insbesondere einen Hall-Sensor, zum Bereitstellen des Messsignals umfasst.

**15.** Pumpensystem (100) nach einem der Ansprüche 12 bis 14, wobei die Lagerwirkrichtung im Wesentlichen parallel zur Rotationsachse (X) angeordnet ist.

**16.** Verfahren zum Ansteuern einer Blutpumpe (300),

wobei die Blutpumpe (300) einen in einem Gehäuse (301) magnetisch gelagerten und zum Fördern eines Fluids um eine Rotationsachse (X) rotierbaren Rotor (320) und einen Stator (340) umfasst, wobei der Stator (340) eingerichtet ist zum Erzeugen eines variierbaren Statormagnetfelds zum Ausüben einer gemäß einem Lagersteuersignal variierbaren Lagerkraft entlang einer Lagerwirkrichtung auf den Rotor (320);

wobei die Blutpumpe (300) eingerichtet ist zum Bereitstellen eines von einer Lagerposition des Rotors (320) entlang der Lagerwirkrichtung abhängigen Messsignals, das einen von einer Rotation des Rotors (320) um die Rotationsache (X) und/oder einer Lateralbewegung des Rotors (320) senkrecht zur Lagerwirkrichtung abhängigen Störanteil umfasst;

wobei das Verfahren umfasst:

Erfassen des Messsignals,

Bestimmen eines von dem Störanteil abhängigen Störsignals basierend auf dem in einem Erfassungsintervall erfassten Messsignal,

Bestimmen, basierend auf dem Messsignal und dem bestimmten Störsignal, einer Vorgabe für das Lagersteuersignal derart, dass der Rotor (320) mittels der Lagerkraft berührungsfrei in dem Gehäuse (301) gelagert wird, wobei eine zum Ausüben der Lagerkraft aufgenommene Leistung gegenüber einer ohne Berücksichtigung des Störsignals bestimmbaren Vorgabe für das Lagersteuersignal reduziert ist, und zeitlich nach dem Erfassungsintervall erfolgendes Ansteuern des Stators (340) gemäß dem Lagersteuersignal entsprechend der bestimmten Vorgabe.

## Fig. 1

## Fig. 2

## Fig. 3

# Fig. 4a

# Fig. 4b

# Fig. 4c

# Fig. 5

# Fig. 6a

# Fig. 6b

**Europäisches Patentamt**
**European Patent Office**
**Office européen des brevets**

# EUROPÄISCHER RECHERCHENBERICHT

**Nummer der Anmeldung**

**EP 22 16 3251**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 6 447 265 B1 (ANTAKI JAMES F [US] ET AL) 10. September 2002 (2002-09-10) | 1-12,14, 15 | INV. A61M60/122 |
| A | * Spalte 6, Zeile 49 – Spalte 9, Zeile 15; Abbildungen 1-5 * | 13 | A61M60/216 A61M60/422 A61M60/538 |
| | ----- | | A61M60/822 |
| A | US 2012/095280 A1 (TIMMS DANIEL [AU]) 19. April 2012 (2012-04-19) * Absätze [0166] – [0259]; Abbildungen 1-19 * | 1 | |
| | ----- | | |
| A | US 5 928 131 A (PREM EDWARD K [US]) 27. Juli 1999 (1999-07-27) * Spalte 4, Zeile 61 – Spalte 11, Zeile 10; Ansprüche 1-7; Abbildungen 1-6 * | 1 | |
| | ----- | | |

**RECHERCHIERTE SACHGEBIETE (IPC)**

**A61M**

Der vorliegende Recherchenbericht wurde für alle Patentansprüche erstellt

| Recherchenort | Abschlußdatum der Recherche | Prüfer |
|---|---|---|
| Den Haag | 6. Dezember 2022 | Schlaug, Martin |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

..........................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 22 16 3251

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-12-2022

| Im Recherchenbericht angeführtes Patentdokument | Datum der Veröffentlichung | Mitglied(er) der Patentfamilie | Datum der Veröffentlichung |
|---|---|---|---|
| US 6447265 B1 | 10-09-2002 | AT 206937 T | 15-11-2001 |
| | | AT 303823 T | 15-09-2005 |
| | | AU 727084 B2 | 30-11-2000 |
| | | BR 9709969 A | 11-01-2000 |
| | | CA 2259642 A1 | 31-12-1997 |
| | | DE 69707470 T2 | 16-05-2002 |
| | | DE 69734165 T2 | 29-06-2006 |
| | | EP 0914171 A2 | 12-05-1999 |
| | | EP 1114648 A2 | 11-07-2001 |
| | | ES 2153312 A1 | 16-02-2001 |
| | | JP 2001514532 A | 11-09-2001 |
| | | US 6015272 A | 18-01-2000 |
| | | US 6447265 B1 | 10-09-2002 |
| | | WO 9749440 A2 | 31-12-1997 |
| US 2012095280 A1 | 19-04-2012 | AU 2010237614 A1 | 20-10-2011 |
| | | CN 102458498 A | 16-05-2012 |
| | | EP 2419160 A1 | 22-02-2012 |
| | | JP 5719829 B2 | 20-05-2015 |
| | | JP 2012523856 A | 11-10-2012 |
| | | JP 2012523857 A | 11-10-2012 |
| | | US 2012095280 A1 | 19-04-2012 |
| | | WO 2010118476 A1 | 21-10-2010 |
| US 5928131 A | 27-07-1999 | CA 2251322 A1 | 26-05-1999 |
| | | DE 19854724 A1 | 27-05-1999 |
| | | FR 2771295 A1 | 28-05-1999 |
| | | FR 2802428 A1 | 22-06-2001 |
| | | FR 2802429 A1 | 22-06-2001 |
| | | GB 2335146 A | 15-09-1999 |
| | | IT RM980721 A1 | 25-05-2000 |
| | | JP 3182402 B2 | 03-07-2001 |
| | | JP H11241695 A | 07-09-1999 |
| | | NL 1010651 C2 | 06-04-2000 |
| | | US 5928131 A | 27-07-1999 |
| | | US 6179773 B1 | 30-01-2001 |
| | | US 6363276 B1 | 26-03-2002 |
| | | US 6375607 B1 | 23-04-2002 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **YOUQING WANG ; FURONG GAO ; FRANCIS J. DOYLE.** Survey on iterative learning control, repetitive control, and run-to-run control. *Journal of Process Control,* 2009, vol. 19 (10), ISSN 0959-1524, 1589-1600

- **DOUGLAS A. BRISTOW ; MARINA THARAYIL ; ANDREW G. ALLEYNE.** Survey Of Iterative Learning Control : A Learning-Based Method for High-Performance Tracking Control. *IEEE Control Systems,* 2006, vol. 26 (3), ISSN 1066-033X, 96-114